# EUROPEAN PATENT APPLICATION

(11) **EP 3 485 858 A1**
(43) Date of publication of application: **22.05.2019**
(21) Application number: 18754305.3
(22) Date of filing: 12.02.2018
(51) Int. Cl.: A61F 13/02, A61F 13/00

(54) **MEDICAL DRESSING MATERIAL**

(30) Priority: 15.02.2017 KR 20170020663
(71) Applicant: Park, Young Joon, Yangsan-si, Gyeongsangnam-do 50515 (KR)
(72) Inventor: Park, Young Joon, Yangsan-si, Gyeongsangnam-do 50515 (KR)
(74) Representative: Mammel und Maser
(86) International application number: PCT/KR2018/001790
(87) International publication number: WO 2018/151474

(57) **Abstract**

The present disclosure relates to a medical dressing patch which is used to simultaneously protect and treat an injury to a skin such as burns, wounds, and traumas, and more particularly, to a medical dressing patch including: a release paper; and an adhesive sheet which is fixed to an upper portion of the release paper and attached to a skin, in which the release paper includes a fixing release paper, and a separation release paper which is fixed to the fixing release paper through a cut-out portion and a connecting portion formed on the fixing release paper, and positioned such that a predetermined region of the separation release paper overlaps a lower surface of the adhesive sheet so that the separation release paper is separated together with the adhesive sheet when the adhesive sheet is separated from the fixing release paper.

## Description

### BACKGROUND

### Field

The present disclosure relates to a medical dressing patch which is used to simultaneously protect and treat an injury to a skin such as burns, wounds, and traumas, and more particularly, to a medical dressing patch which includes an adhesive sheet which is to be attached to a skin, a separation release paper which is used to separate the adhesive sheet and attach the adhesive sheet to the skin, and a fixing release paper which fixes the separation release paper and the adhesive sheet so that the separation release paper and the adhesive sheet are stored, thereby improving convenience for use and storage performance.

### Description of the Related Art

In general, a medical dressing patch is widely used to simultaneously protect and treat a slight injury to a skin such as burns, wounds, and traumas. That is, the medical dressing patch serves to prevent further contamination by reducing heat vaporization and a loss of moisture from a wound surface, and serves to reduce a growth of bacteria existing in the wound.

The medical dressing patch has a configuration in which an adhesive sheet is attached to an upper surface of a release paper, and provides the aforementioned functions as the adhesive sheet is separated from the release paper and an attachment surface formed at a lower side of the adhesive sheet is attached to a wound site.

A user holds one side of the adhesive sheet to separate the adhesive sheet from the release paper, takes the adhesive sheet off the release paper, and then attaches the adhesive sheet to the wound site. However, in this case, the adhesive sheet may be contaminated because the user holds the adhesive sheet. Moreover, a thickness of the adhesive sheet tends to be gradually decreased to further ensure wearing comfort and activity of a patient with the attached adhesive sheet, and as a result, an operation of separating the adhesive sheet from the release paper causes inconvenience to the user, and thus convenience for use deteriorates.

As a related art for solving the problem, there is Korean Utility Model Registration No. 439156. The related art has a main configuration in which a user easily separates a protective sheet from a release paper by holding a handle portion of a separation release paper, attaches the protective sheet to a wound site, and then separates the separation release paper from the protective sheet.

With this configuration, the user may separate the protective sheet from the release paper by holding the handle portion of the separation release paper without holding the protective sheet, and then may attach the protective sheet to the wound site, and as a result, it is possible to expect an effect of improving convenience and preventing secondary bacterial infection.

However, the handle portion has a small area, and an attachment surface attached to the protective sheet is large, and as a result, it is very difficult for the user to hold the handle portion by hand. In addition, the handle portion is frequently withdrawn during a process of holding and taking off the handle portion. To prevent the withdrawal of the handle portion, the user needs to hold the handle portion and very carefully peel off the separation release paper attached to the protective sheet, and as a result, a large amount of time is required to separate the protective sheet and then attach the protective sheet to the wound site. Moreover, in this process, the user's hand comes into contact with an adhesive portion of the protective sheet, which may cause secondary bacterial infection.

Meanwhile, recently, a demand for a hydrocolloid-based adhesive sheet, which have an excellent ability to protect a wound and excellent adhesion, is increased. The hydrocolloid adhesive sheet is advantageous in terms of wearing comfort and activity of a patient because the hydrocolloid adhesive sheet is transparent and has a small thickness, and the hydrocolloid adhesive sheet is also advantageous in terms of beauty because the hydrocolloid adhesive sheet is hardly recognized with the naked eye even though the hydrocolloid adhesive sheet is attached to a skin. However, because of properties of the transparent and thin hydrocolloid adhesive sheet, there is a problem in that it is very difficult for the user to separate the hydrocolloid adhesive sheet from a release paper.

### [Document of Related Art]

### [Patent Document]

Korean Utility Model Registration No. 439156

### SUMMARY

The present disclosure has been made in an effort to solve the aforementioned problems, and an object of the present disclosure is to provide a medical dressing patch capable of allowing an adhesive sheet to be easily separated from a release paper, and allowing a separation release paper to be easily separated from the adhesive sheet after the adhesive sheet is attached to a wound site, thereby improving convenience for use and preventing secondary bacterial infection.

According to an aspect of the present disclosure, there is provided a medical dressing patch including: a release paper 200; and an adhesive sheet 100 which is fixed to an upper portion of the release paper 200 and attached to a skin, in which the release paper 200 includes a fixing release paper 210, and a separation release paper 220 which is fixed to the fixing release paper 210 through a cut-out portion 211 and a connecting portion 212 formed on the fixing release paper 210, and positioned such that a predetermined region of the separation release paper 220 overlaps a lower surface of the adhesive sheet 100 so that the separation release paper 220 is separated together with the adhesive sheet 100 when the adhesive sheet 100 is separated from the fixing release paper 210.

Here, the adhesive sheet 100 may have a circular shape, and the separation release paper 220 may have a shape in which two circular portions are spaced apart from each other at a predetermined interval and connected to each other so as to form depressed portions 221.

In addition, a center point of the adhesive sheet 100 and center points of the two circular portions of the separation release paper 220 may be disposed to be positioned on an imaginary identical extension line.

Further, one or more first identification portions 213, which are selected from words, patterns, and figures so that whether the adhesive sheet 100 is positioned on an upper surface of the separation release paper 220 is visually checked, may be provided at a predetermined position of the fixing release paper 210.

In addition, in the medical dressing patch according to the present disclosure, a third identification portion 223, which is configured by a protrusion so that whether the adhesive sheet 100 is positioned on an upper surface of the separation release paper 220 is tactilely checked, may be formed at predetermined position of an upper surface of the separation release paper 220.

Further, the fixing release paper 210 and the separation release paper 220 may have a thickness of 20 to 300 µm so as to have predetermined restoring force, the cut-out portion 211 may be formed along an outer circumferential edge portion of the separation release paper 220, two connecting portions 212 may be formed in the cut-out portion 211 at an outer circumferential edge portion of the other circular portion that does not overlap the adhesive sheet 100, and the connecting portions 212 may be positioned to face each other at a position perpendicularly intersecting the imaginary extension line while running through the center point of the circular portion.

In addition, the adhesive sheet 100 may have a circular shape, the separation release paper 220 may have a shape in which two circular portions are spaced apart from each other at a predetermined interval and connected to each other so as to form depressed portions 221, a center point of the adhesive sheet 100 and center points of the two circular portions of the separation release paper 220 may be disposed to be positioned on an imaginary identical extension line, one or more first identification portions 213, which are selected from words, patterns, and figures so that whether the adhesive sheet 100 is positioned on an upper surface of the separation release paper 220 is visually checked, may be provided at a predetermined position of the fixing release paper 210, a third identification portion 223, which is configured by a protrusion so that whether the adhesive sheet 100 is positioned on the upper surface of the separation release paper 220 is tactilely checked, may be formed at predetermined position of the upper surface of the separation release paper 220, the fixing release paper 210 and the separation release paper 220 may have a thickness of 20 to 300 µm so as to have predetermined restoring force, the cut-out portion 211 may be formed along an outer circumferential edge portion of the separation release paper 220, two connecting portions 212 may be formed in the cut-out portion 211 at an outer circumferential edge portion of the other circular portion that does not overlap the adhesive sheet 100, and the connecting portions 212 may be positioned to face each other at a position perpendicularly intersecting the imaginary extension line while running through the center point of the circular portion.

According to another aspect of the present disclosure, there is provided a medical dressing patch including: a single sheet of release paper 200 having a predetermined size; and a plurality of adhesive sheets 100 which is fixed to an upper portion of the release paper 200 and attached to a skin, in which the release paper 200 includes a fixing release paper 210 which is separated from the adhesive sheets 100, and separation release papers 220 which are fixed to the fixing release paper 210 through cut-out portions 211 and connecting portions 212 formed on the fixing release paper 210, and positioned such that predetermined regions of the separation release papers 220 overlap lower surfaces of the adhesive sheets 100 so that the separation release paper 220 is separated together with the adhesive sheet 100 when the adhesive sheet 100 is separated from the fixing release paper 210, and the number of separation release papers 220 is equal to the number of adhesive sheets 100.

### BRIEF DESCRIPTION OF THE DRAWINGS

The above and other aspects, features and other advantages of the present disclosure will be more clearly understood from the following detailed description taken in conjunction with the accompanying drawings, in which:
FIG. 1 is a perspective view of a medical dressing patch according to a first exemplary embodiment of the present disclosure;
FIG. 2 is an exploded perspective view of the medical dressing patch illustrated in FIG. 1;
FIG. 3 is a perspective view of a medical dressing patch according to a second exemplary embodiment of the present disclosure;
FIGS. 4 and 5 are perspective views for explaining a method of using the medical dressing patch according to the present disclosure; and

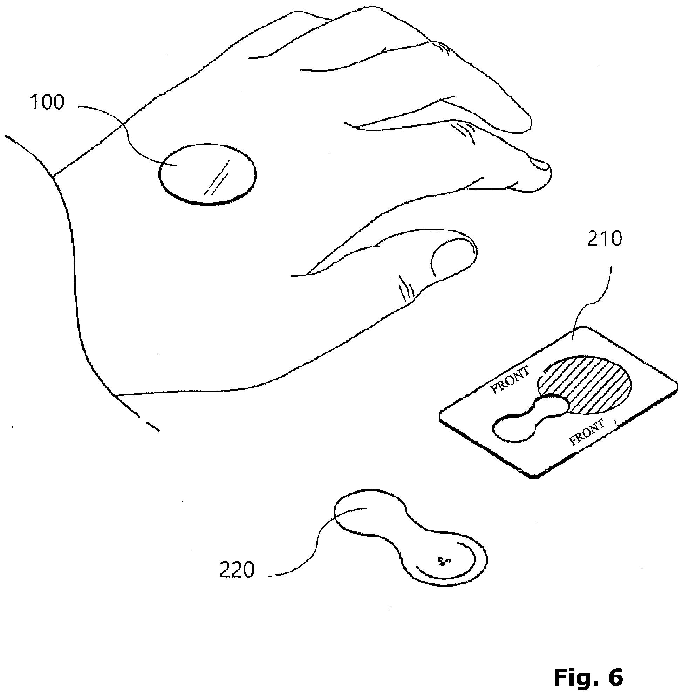

FIG. 6 is a perspective view of the medical dressing patch according to the present disclosure after use.

### DETAILED DESCRIPTION OF THE PREFERRED EMBODIMENT

In the present application, it will be appreciated that term "including", "having", or "comprising" is intended to indicate the presence of characteristics, numbers, steps, constituent elements, and components described in the specification or a combination thereof, and does not pre-exclude a possibility of the presence or addition of one or more other characteristics, numbers, steps, operations, constituent elements, and components, or a combination thereof.

Hereinafter, a medical dressing patch according to the present disclosure will be described with reference to the accompanying drawings. In the drawings, like reference numerals indicate like constituent elements, and a duplicated description of like constituent elements will be omitted.

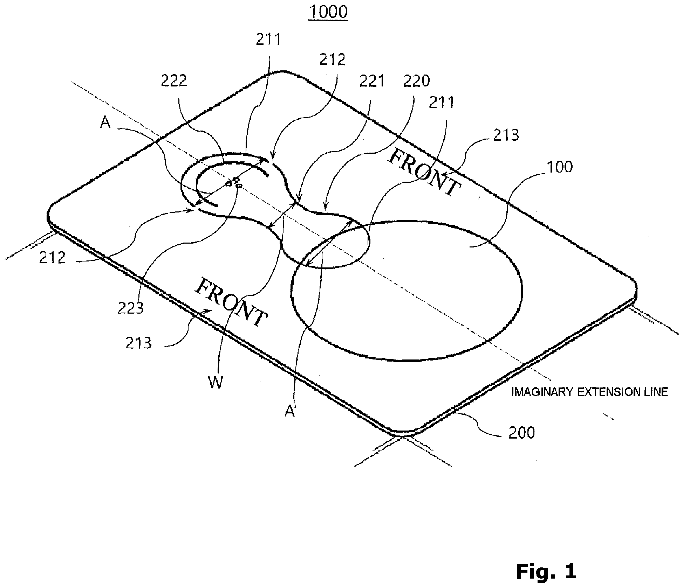

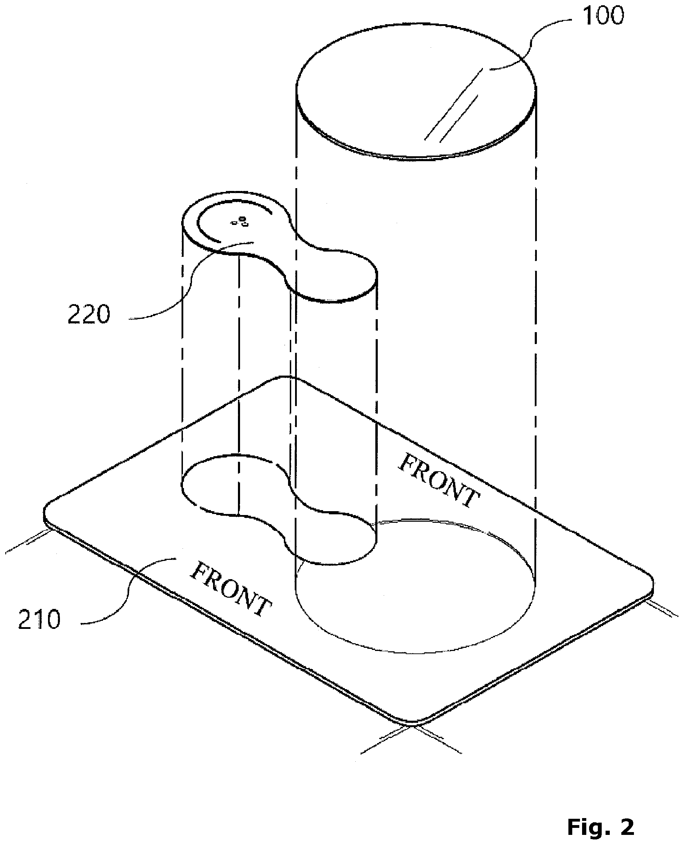

FIG. 1 is a perspective view of a medical dressing patch according to a first exemplary embodiment of the present disclosure, and FIG. 2 is an exploded perspective view of the medical dressing patch illustrated in FIG. 1.

As illustrated in FIGS. 1 and 2, a medical dressing patch 1000 according to a first exemplary embodiment of the present disclosure includes a release paper 200 and an adhesive sheet 100.

The release paper 200 will be specifically described first. The release paper 200 includes a fixing release paper 210 which is to be separated from the adhesive sheet 100, and a separation release paper 220 which is to be separated together with the adhesive sheet 100, and a smooth coating layer may be formed on at least one surface of the release paper 200.

Cut-out portions 211, which have a shape identical to an external shape of the separation release paper 220, are provided in the fixing release paper 210, such that the separation release paper 220 is easily separated from the fixing release paper 210 when the adhesive sheet 100 is used, and at normal times, a state in which the separation release paper 220 and the fixing release paper 210 are connected to each other by connecting portions 212 formed in the cut-out portions 211 is maintained.

Here, one or more first identification portions 213, which are selected from words, patterns, and figures, may be provided at a predetermined position of the fixing release paper 210.

In the present disclosure, a part of a lower surface of the adhesive sheet 100 is attached to the separation release paper 220, and the remaining part of the lower surface of the adhesive sheet 100 is attached to the fixing release paper 210. Therefore, when a user separates the separation release paper 220 in order to use the adhesive sheet 100, the user needs to draw the separation release paper 220 toward a portion, where the adhesive sheet 100 is attached, in order to separate the separation release paper 220 in a state in which the adhesive sheet 100 is attached to the separation release paper 220. If the user draws the separation release paper 220 in the opposite direction, only the separation release paper 220 is separated from the adhesive sheet 100, such that it may be difficult to use the adhesive sheet 100.

In particular, recently, the release paper and the adhesive sheet, which constitute the dressing patch, have high transparency, and as a result, in many instances, it is difficult for the user to easily recognize which surface of the separation release paper 220 the adhesive sheet 100 is attached to. Moreover, in a case in which the user has poor eyesight, it is very much difficult for the user to recognize where the adhesive sheet 100 is attached.

Therefore, in the present disclosure, the first identification portion 213 such as words, patterns, and figures is formed on the fixing release paper 210, thereby allowing the user to easily recognize which direction the user needs to draw the separation release paper 220.

As described above, the separation release paper 220 is fixed to the fixing release paper 210 through the cut-out portions 211 and the connecting portions 212 formed on the fixing release paper 210, and a part of the separation release paper 220 is positioned to overlap the lower surface of the adhesive sheet 100 so that the separation release paper 220 may be separated together with the adhesive sheet 100 when the adhesive sheet 100 is separated from the fixing release paper 210.

In addition, the separation release paper 220 includes two circular portions spaced apart from each other at a predetermined interval, and depressed portions 221 having a predetermined radius of curvature may be formed between the circular portions so that the separation release paper 220 may be easily separated when the separation release paper 220 is separated. Specifically, a width W formed by the depressed portions 221 is smaller than a diameter A or A' of each of the two circular portions. With this shape, the user may hold the circular portion having a large area by hand, such that the user may easily hold the separation release paper 220, and the user may softly draw the separation release paper 220 because the depressed portions 221 having a relatively small width W are provided.

Moreover, a center point of the adhesive sheet 100 and center points of the two circular portions, which constitute the separation release paper 220, may be disposed to be positioned on an imaginary extension line. The reason is to take the separation release paper 220 off the fixing release paper 210 in a state in which the adhesive sheet 100 is not twisted or biased toward any one side when the separation release paper 220 is separated from the fixing release paper 210.

Meanwhile, a second identification portion 222 and/or a third identification portion may be formed at a predetermined position of the separation release paper 220.

Specifically, the second identification portion 222 performs the same function as the first identification portion 213 that allows the user to easily recognize which surface of the separation release paper 220 the adhesive sheet 100 is attached to as described above, and the second identification portion 222 helps the user to easily and visually recognize which part the user needs to hold.

In particular, the second identification portion 222 serves to prevent the situation in which the user cannot quickly use the adhesive sheet 100 because it is very difficult for the user to recognize, with the naked eye, which part of the release paper 200 the separation release paper 220 is positioned at in a case in which the fixing release paper 210 and the separation release paper 220 are made of the same transparent material.

Here, the second identification portion 222 may be one or more items selected from patterns, words, and figures, and as an example, as illustrated in FIGS. 1 and 2, a band may be formed along the cut-out portion 211 at a predetermined position of an inner surface of the separation release paper 220.

The third identification portion 223 performs the same function as the first identification portion 213 that allows the user to easily recognize which surface of the separation release paper 220 the adhesive sheet 100 is attached to as described above, and the third identification portion 223 helps the user to easily and tactilely recognize which part the user needs to hold.

That is, the third identification portion 223 serves to prevent the situation in which the user cannot quickly use the adhesive sheet 100 because it is difficult for the user to recognize, with the naked eye, which part of the release paper 200 the separation release paper 220 is positioned at.

Here, as illustrated in FIGS. 1 and 2, the third identification portion 223 may be configured by one or more protrusions positioned at a predetermined position at an inner upper side of the separation release paper 220 which is adjacent to the cut-out portion 211.

Meanwhile, the fixing release paper 210 and the separation release paper 220 may be transparent or opaque by being made of a plastic material such as polyethylene terephthalate PET or polyethylene PE, and may have a thickness of 20 to 300 µm so as to have predetermined restoring force so that the fixing release paper 210 and the separation release paper 220 may be quickly restored to the original position even in a case in which the fixing release paper 210 and the separation release paper 220 are bent. If the thickness is below 20 µm, the thickness is too small, such that restoring force is insufficient. If the thickness is above 300 µm, the fixing release paper 210 and the separation release paper 220 are not bent well, such that the separation release paper 220 is not easily separated. Accordingly, the fixing release paper 210 and the separation release paper 220 may have the aforementioned thickness.

In addition, the two connecting portions 212 may be formed in the cut-out portions 211 at an outer circumferential edge portion of one circular portion, which does not overlap the adhesive sheet 100, so as to allow the separation release paper 220 to be easily separated and prevent the separation release paper 220 from coming off the fixing release paper 210.

Specifically, as illustrated in FIG. 1, the two connecting portions 212 may be provided to face each other at a position perpendicularly intersecting the imaginary extension line that runs through the center points of the two circular portions of the separation release paper 220 and the center point of the adhesive sheet 100. One circular portion of the separation release paper 220 is positioned under the adhesive sheet 100 and fixed by the adhesive sheet 100, and the other circular portion of the separation release paper 220 is fixed by the two connecting portions 212, and as a result, it is possible to not only quickly separate the separation release paper 220, but also prevent the separation release paper 220 from coming off the fixing release paper 210 in the vicinity of the cut-out portion 211 by restoring force of the fixing release paper 210 and the separation release paper 220.

Next, between the two circular portions of the separation release paper 220, the circular portion on which the identification portions 213, 222, and 223 are formed may have a larger cross-sectional area than the circular portion which is in contact with the lower surface of the adhesive sheet 100. The reason is to allow the user to easily hold the separation release paper 220 and to easily separate the separation release paper 220 after the adhesive sheet 100 is attached to the skin.

Moreover, an area of the circular portion of the separation release paper 220, which is attached to the adhesive sheet 100, may be 10% to 50%, more particularly, 20% to 40% of an overall area of the adhesive sheet 100. If the circular portion of the separation release paper 220 is attached to the adhesive sheet 100 with an area below 10%, the adhesive force is too low, such that the adhesive sheet 100 may not be separated together with the separation release paper 220 when the separation release paper 220 is separated. On the contrary, if the circular portion of the separation release paper 220 is attached to the adhesive sheet 100 with an area above 50%, the adhesive force is too high, such that the separation release paper 220 is not separated well from the adhesive sheet 100 after the adhesive sheet 100 is attached to the skin. Accordingly, the separation release paper 220 and the adhesive sheet 100 may be attached to each other within the aforementioned range.

Meanwhile, the adhesive sheet 100 may be a hydrocolloid adhesive sheet, or a polyurethane foam adhesive sheet, the hydrocolloid adhesive sheet has a form in which hydrophilic hydrocolloid polymers are dispersed on a hydrophobic rubber matrix attachment surface having adhesion, and the polyurethane foam adhesive sheet has a form in which polymers such as polyethylene glycol, isocyanate, catalysts, and water are mixed and dispersed on a hydrophobic urethane attachment surface having adhesion. Because the hydrocolloid adhesive sheet or the polyurethane foam adhesive sheet is a sheet which is widely used because this sheet has an ability to protect a wound based on hygroscopicity and wet environment maintenance and has excellent adhesion, a more specific description thereof will be omitted.

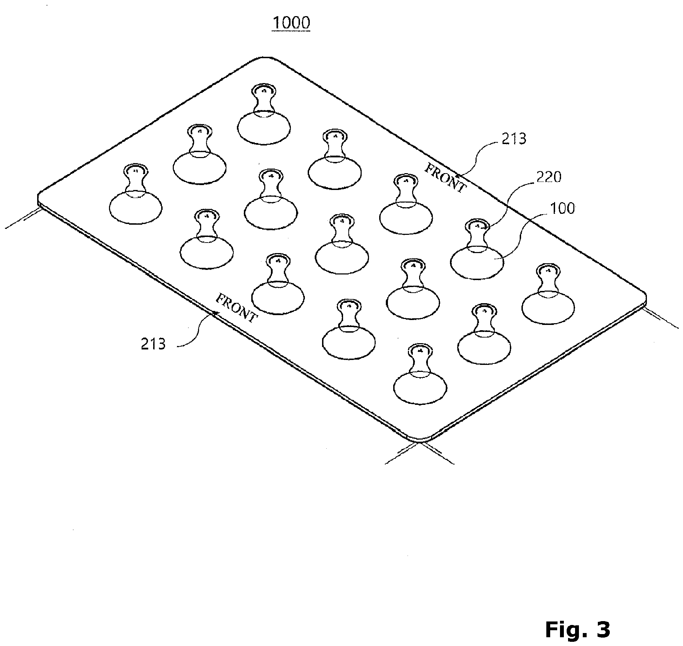

Hereinafter, a medical dressing patch according to a second exemplary embodiment of the present disclosure will be described with reference to FIG. 3.

The second exemplary embodiment is identical to the first exemplary embodiment, which is described with reference to FIGS. 1 and 2, except for the number of adhesive sheets 100, the number of separation release papers 220, and an arrangement structure of the adhesive sheets 100 and the separation release papers 220. Therefore, like reference numerals indicate like constituent elements, and a repeated description of like constituent elements will be omitted.

The second exemplary embodiment includes a single sheet of release paper 200 and a plurality of adhesive sheets 100.

As an example, as illustrated in FIG. 3, a total of fifteen adhesive sheets 100 may be provided on an upper surface of the single release paper 200 having predetermined width and area, in which three adhesive sheets 100 are disposed in each row, and five adhesive sheets 100 are disposed in each column perpendicular to the row. In addition, the aforementioned separation release paper 220 is provided on the bottom surface of each of the adhesive sheets 100.

Here, the separation release papers 220 may be disposed to be inclined at a predetermined angle so that more adhesive sheets 100 may be attached per unit area of the release paper 200. Specifically, assuming that the row in which the three adhesive sheets 100 are provided is a horizontal row and the column in which the five adhesive sheets 100 are provided is a vertical column, the separation release paper 220 is disposed to be inclined at 40° to 50° with respect to the horizontal row and the vertical column, and as a result, it is possible to maximize an utilization area of the fixing release paper 210.

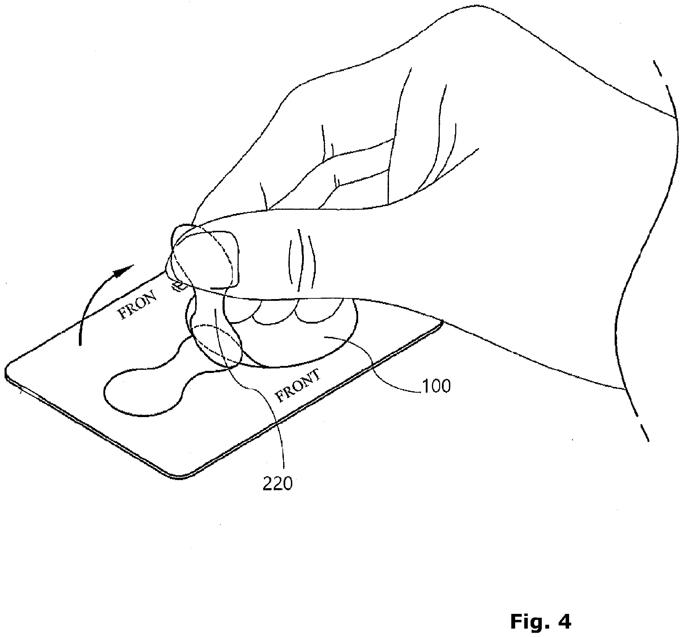

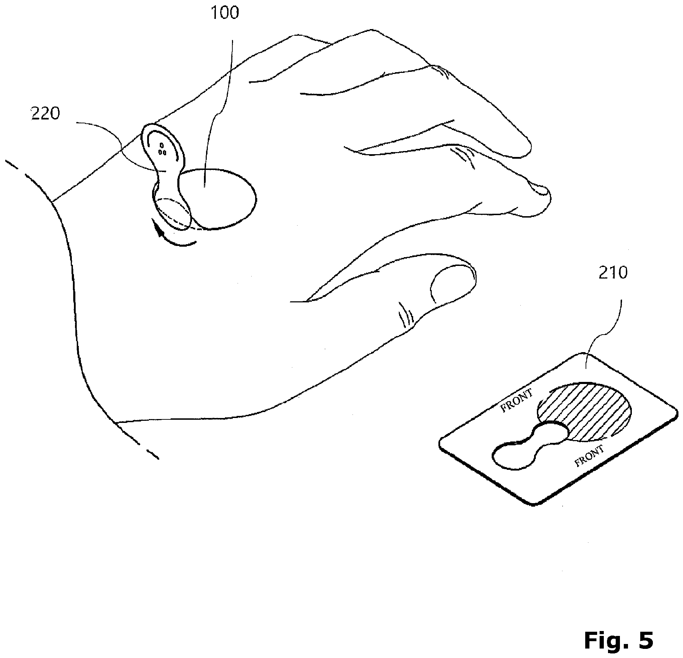

A method of using the medical dressing patch according to the present disclosure will be described with reference to FIGS. 4 and 5.

As illustrated in FIG. 4, first, to separate the adhesive sheet 100 attached to one surface of the release paper 200, the user checks whether the adhesive sheet 100 exists on the upper surface of the release paper 200 by recognizing any one of the first identification portion 213, the second identification portion 222, and the third identification portion 223. Next, the user draws the separation release paper 220 upward or draws the fixing release paper 210 downward, and takes the separation release paper 220 off the upper surface of the fixing release paper 210 while holding a predetermined portion of the separation release paper 220 separated along a cut-out line. Then, the adhesive sheet 100, to which a predetermined portion of the separation release paper 220 is attached, is separated together.

Next, as illustrated in FIG. 5, the user attaches the adhesive sheet 100 to a wound site while holding the separation release paper 220, and takes the separation release paper 220 off the adhesive sheet 100.

As is apparent from the above description, in the medical dressing patch 1000 according to the present disclosure, it is possible to separate the adhesive sheet 100 from the release paper 200 via the separation release paper 220 and then to attach the adhesive sheet 100 to the skin without touching the adhesive sheet 100, whereby the adhesive sheet 100 is prevented from being contaminated during the attachment thereof. In addition, a predetermined portion of the separation release paper 220, which is partially separated, can be held in order to repeatedly perform the separation and attachment of the adhesive sheet. Consequently, the rapid use of the medical dressing patch is possible.

Although the specific parts of the present disclosure have been described in detail, it will be obvious to those skilled in the art that such a specific description is just a preferred embodiment and the scope of the present disclosure is not limited thereby, and it will be apparent to those skilled in the art that various alterations and modifications are possible within the scope and technical spirit of the present disclosure, and it is natural that such alterations and modifications also fall within the accompanying claims.

### Industrial Applicability

In the medical dressing patch according to the present disclosure, the separation release paper is attached to a predetermined portion of the adhesive sheet, and the separation release paper is fixed or separated by the cut-out portion and the connecting portion formed on the fixing release paper. Consequently, the medical dressing patch according to the present disclosure is advantageous in that it is possible to rapidly use the adhesive sheet.

In addition, the medical dressing patch according to the present disclosure includes a plurality of identification portions, which include patterns, words, figures, and protrusions. Consequently, the medical dressing patch according to the present disclosure is advantageous in that it is possible to rapidly and accurately check the position at which the separation release paper is held and the direction in which the adhesive sheet is separated, whereby it is possible to increase accuracy in using the medical dressing patch.

## Claims

1. A medical dressing patch comprising:
a release paper 200; and
an adhesive sheet 100 which is fixed to an upper portion of the release paper 200 and attached to a skin,
wherein the release paper 200 includes a fixing release paper 210, and a separation release paper 220 which is fixed to the fixing release paper 210 through a cut-out portion 211 and a connecting portion 212 formed on the fixing release paper 210, and positioned such that a predetermined region of the separation release paper 220 overlaps a lower surface of the adhesive sheet 100 so that the separation release paper 220 is separated together with the adhesive sheet 100 when the adhesive sheet 100 is separated from the fixing release paper 210.

2. The medical dressing patch according to claim 1, wherein the adhesive sheet 100 has a circular shape, and the separation release paper 220 has a shape in which two circular portions are spaced apart from each other at a predetermined interval and connected to each other so as to form depressed portions 221.

3. The medical dressing patch according to claim 2, wherein a center point of the adhesive sheet 100 and center points of the two circular portions of the separation release paper 220 are disposed to be positioned on an imaginary identical extension line.

4. The medical dressing patch according to claim 1, wherein one or more first identification portions 213, which are selected from words, patterns, and figures so that whether the adhesive sheet 100 is positioned on an upper surface of the separation release paper 220 is visually checked, are provided at a predetermined position of the fixing release paper 210.

5. The medical dressing patch according to claim 1, wherein a third identification portion 223, which is configured by a protrusion so that whether the adhesive sheet 100 is positioned on an upper surface of the separation release paper 220 is tactilely checked, is formed at predetermined position of an upper surface of the separation release paper 220.

6. The medical dressing patch according to claim 3, wherein the fixing release paper 210 and the separation release paper 220 have a thickness of 20 to 300 µm so as to have predetermined restoring force, the cut-out portion 211 is formed along an outer circumferential edge portion of the separation release paper 220, two connecting portions 212 are formed in the cut-out portion 211 at an outer circumferential edge portion of the other circular portion that does not overlap the adhesive sheet 100, and the connecting portions 212 are positioned to face each other at a position perpendicularly intersecting the imaginary extension line while running through the center point of the circular portion.

7. The medical dressing patch according to claim 1, wherein the adhesive sheet 100 has a circular shape, the separation release paper 220 has a shape in which two circular portions are spaced apart from each other at a predetermined interval and connected to each other so as to form depressed portions 221, a center point of the adhesive sheet 100 and center points of the two circular portions of the separation release paper 220 are disposed to be positioned on an imaginary identical extension line, one or more first identification portions 213, which are selected from words, patterns, and figures so that whether the adhesive sheet 100 is positioned on an upper surface of the separation release paper 220 is visually checked, are provided at a predetermined position of the fixing release paper 210, a third identification portion 223, which is configured by a protrusion so that whether the adhesive sheet 100 is positioned on the upper surface of the separation release paper 220 is tactilely checked, is formed at predetermined position of the upper surface of the separation release paper 220, the fixing release paper 210 and the separation release paper 220 have a thickness of 20 to 300 µm so as to have predetermined restoring force, the cut-out portion 211 is formed along an outer circumferential edge portion of the separation release paper 220, two connecting portions 212 are formed in the cut-out portion 211 at an outer circumferential edge portion of the other circular portion that does not overlap the adhesive sheet 100, and the connecting portions 212 are positioned to face each other at a position perpendicularly intersecting the imaginary extension line while running through the center point of the circular portion.

8. A medical dressing patch comprising:
a single sheet of release paper 200 having a predetermined size; and
a plurality of adhesive sheets 100 which is fixed to an upper portion of the release paper 200 and attached to a skin,
wherein the release paper 200 includes a fixing release paper 210 which is separated from the adhesive sheets 100, and separation release papers 220 which are fixed to the fixing release paper 210 through cut-out portions 211 and connecting portions 212 formed on the fixing release paper 210, and positioned such that predetermined regions of the separation release papers 220 overlap lower surfaces of the adhesive sheets 100 so that the separation release paper 220 is separated together with the adhesive sheet 100 when the adhesive sheet 100 is separated from the fixing release paper 210, and the number of separation release papers 220 is equal to the number of adhesive sheets 100.

9. The medical dressing patch according to claim 8, wherein the adhesive sheet 100 has a circular shape, the separation release paper 220 has a shape in which two circular portions are spaced apart from each other at a predetermined interval and connected to each other so as to form depressed portions 221, a center point of the adhesive sheet 100 and center points of the two circular portions of the separation release paper 220 are disposed to be positioned on an imaginary identical extension line, one or more first identification portions 213, which are selected from words, patterns, and figures so that whether the adhesive sheet 100 is positioned on an upper surface of the separation release paper 220 is visually checked, are provided at a predetermined position of the fixing release paper 210, a third identification portion 223, which is configured by a protrusion so that whether the adhesive sheet 100 is positioned on the upper surface of the separation release paper 220 is tactilely checked, is formed at predetermined position of the upper surface of the separation release paper 220, the fixing release paper 210 and the separation release paper 220 have a thickness of 20 to 300 µm so as to have predetermined restoring force, the cut-out portion 211 is formed along an outer circumferential edge portion of the separation release paper 220, two connecting portions 212 are formed in the cut-out portion 211 at an outer circumferential edge portion of the other circular portion that does not overlap the adhesive sheet 100, and the connecting portions 212 are positioned to face each other at a position perpendicularly intersecting the imaginary extension line while running through the center point of the circular portion.
